# EUROPÄISCHE PATENTSCHRIFT

(11) **EP 1 782 845 B1**
(45) Veröffentlichungstag und Bekanntmachung des Hinweises auf die Patenterteilung: **17.12.2008**
(21) Anmeldenummer: 07000986.5
(22) Anmeldetag: 25.04.2003
(51) Int. Cl.: A61L 24/02, A61L 24/04, A61K 49/04

(54) **Knochenzementmischung und Röntgenkontrastmittel sowie Verfahren zu ihrer Herstellung**
Bone cement and X-ray contrast agent and methods of manufacturing the same
Ciment osseux et agent de contraste radiographique et leur procédé de fabrication

(30) Priorität: 29.05.2002 DE 10224346
(43) Veröffentlichungstag der Anmeldung: 09.05.2007
(62) Teilanmeldung aus: 03009377.7
(73) Patentinhaber: Heraeus Kulzer GmbH, 63450 Hanau (DE)
(72) Erfinder: Kühn, Klaus-Dieter, 35041 Marburg (DE)
(74) Vertreter: Kühn, Hans-Christian

(56) Entgegenhaltungen:
- EP-A- 0 041 614
- EP-A- 1 430 913
- WO-A-00/57932
- MISRA D N: "Adsorption of zirconyl salts and their acids on hydroxyapatite: use of the salts as coupling agents to dental polymer composites." JOURNAL OF DENTAL RESEARCH. UNITED STATES DEC 1985, Bd. 64, Nr. 12, Dezember 1985 (1985-12), Seiten 1405-1408, XP009013841 ISSN: 0022-0345
- KIM H Y ET AL: "Improvement of fatigue properties of poly(methyl methacrylate) bone cement by means of plasma surface treatment of fillers" J BIOMED MATER RES;JOURNAL OF BIOMEDICAL MATERIALS RESEARCH 1999 JOHN WILEY & SONS INC, NEW YORK, NY, USA, Bd. 48, Nr. 2, 1999, Seiten 135-142, XP002248232
- SKRTIC DRAGO ET AL: "Physicochemical evaluation of bioactive polymeric composites based on hybrid amorphous calcium phosphates." JOURNAL OF BIOMEDICAL MATERIALS RESEARCH, Bd. 53, Nr. 4, 2000, Seiten 381-391, XP002248233 ISSN: 0021-9304
- KJELLSON F ET AL: "Tensile properties of a bone cement containing non-ionic contrast media" JOURNAL OF MATERIALS SCIENCE: MATERIALS IN MEDICINE 2001 NETHERLANDS, Bd. 12, Nr. 10-12, 2001, Seiten 889-894, XP009013837 ISSN: 0957-4530

## Beschreibung

Die Erfindung betrifft eine Knochenzementmischung, gebildet aus einer ein Röntgenkontrastmittel enthaltenden Polymerkomponente und einer Monomerkomponente, sowie ein Röntgenkontrastmittel. Des weiteren betrifft die Erfindung Verfahren zur Herstellung der Knochenzementmischung und des Räntgenkontrastmittels sowie die Verwendung des Röntgenkontrastmittels.

Unter Knochenzementmischung im Sinne der Erfindung ist ein Zweikomponentensystem zu verstehen, bei dem eine Komponente als pulverförmige Polymerkomponente und eine zweite Komponente als flüssige Monomerkomponente vorliegen. Beide Komponenten werden in der Regel erst unmittelbar vor der Verwendung miteinander gemischt.

Knochenzemente, insbesondere solche, die zur Verankerung künstlicher Gelenke verwendet werden, enthalten Röntgenkontrastmittel zur klinischen Verlaufskontrolle. Diese Röntgenkontrastmittel erlauben dem Chirurgen eine sichere Überprüfung des Implantats und eine frühzeitige Diagnose möglicher Komplikationen, wie z.B. Lockerungen. Knochenzemente sind chemische Kunststoffe auf Acrylatbasis (beispielsweise Palacos^{®} R der Heraeus Kulzer GmbH. Sie werden als Zweikomponenten-Systeme angeboten mit einer pulverförmigen Polymermischung und einem flüssigen Monomer. Als Kontrastmittel werden dabei der Polymermischung Zirkondioxid oder Bariumsulfat zugesetzt. Diese Röntgenkontrastmittel werden allerdings nicht in die Polymerkette eingebunden und gelten deshalb als mögliche Ursache für Mikrorisse im Zementmantel. Zirkondioxid kann an Grenzflächen unter Umständen abrasiv wirken.

EP 41 614 beschreibt beschichtetes BaSO₄-Pulver für Zahnfüllmassen, das durch Dispergieren und Zugabe von Polymerlösung hergestellt wird. EP 89782 beschreibt beschichtetes Bariumsulfat, das durch Mischen von PMMA Teilchen und BaSO₄ Partikeln mit HEMA und anschließendes Erhitzen hergestellt wird. JP 06024927 betrifft Polymer-Füller Composites, die durch Polymerisieren von Säuremonomer und weiterem ungesättigten Monomer in Gegenwart von Röntgenkontrastpulver hergestellt werden.

WO 0057932: Hier werden Composites aus Polymermatrix und festem, flüssigen oder gasförmigen Füller beschrieben, die eine "matrix ligament thickness" von bevorzugt weniger als 250 nm besitzen. Damit ist der Abstand zwischen den Füllerteilchen gemeint. Die

Nanocomposites können hergestellt werden durch Einbringen des Füllers in die Ausgangskomponenten (Precursors) des Zements, Einbringen während des Mischvorgangs der Ausgangskomponenten, Einbringen in eine vorgefertigte Paste, Teig oder Flüssigkeit, wo Polymerisation induziert wird. Die Füller können röntgenopak sein, u.a. Zirkoniumoxid.

Die Beispiele belegen Composites mit folgenden Teilchen: 1-3 Micrometer BaSO₄, 100 nm Ba-SO₄, und 60 nm Al₂O₃ (acrylbeschichtet).

EP 644 780: Anspruch 12 betrifft ein granulatförmiges oder Fasermaterial, bei dem die Füllerpartikel (Größe von 1-15µ) mindestens teilweise von der (Co)Polymermatrix umschlossen sind, wobei die Polymerketten nicht gecrackt sind. Das Material ist durch Mischen und Extrudieren herstellbar.

US 6,080,801 offenbart Polymerkugeln (Fig. 1) mit darin teilweise oder gänzlich enthaltenem Microfüller. Diese Kugeln sind nicht größer als 300 p und enthalten Füllerteilchen der Größe 1-250, bevorzugt 5-15 µ. Hergestellt werden sie z.B. dadurch, dass die Füllerteilchen Kristallisationskeime bei der Polymerisation bilden.

Der Erfindung liegt die Aufgabe zugrunde, ein verbessertes Röntgenkontrastmittel und einen diesen enthaltenden Knochenzement sowie Verfahren zu deren Herstellung zur Verfügung zu stellen.

Erfindungsgemäß wird die Aufgabe durch die Merkmale der unabhängigen Ansprüche gelöst. Das Röntgenkontrastmittel ist ausgebildet
als im Wesentlichen kugelförmige Polymer- oder Copolymerteilchen mit darin dispergierten röntgenopaken anorganischen Nanopartikeln der Teilchengröße 3-15 nm, hergestellt durch Polymerisation, vorzugsweise Suspensionspolymerisation, in Gegenwart der Nanopartikel, wobei die Nanopartikel vollständig von dem (Co)polymermaterial bedeckt sind, und die anorganischen Nanopartikel im Wesentlichen aus ZrO₂ bestehen und mit Silanen Oberflächen modifiziert sind.

Derartige Röntgenkontrastmittel enthaltende Knochenzementmischungen, die eine Polymerkomponente und eine Monomerkomponente aufweisen, wobei die Polymerkomponente Polymere und/oder Copolymere aufweist, werden erfindungsgemäß dadurch hergestellt, dass während der Herstellung der Polymere und/oder Copolymere ein röntgenopake Elemente enthaltendes Polymer und/oder Monomer oder röntgenopake anorganische Nanopartikel der Teilchengröße 3-15 nm zugesetzt werden.

Wenn das Röntgenkontrastmittel röntgenopake anorganische Nanopartikel der Teilchengröße 3-15 nm mit einer äußeren Polymerschicht aufweist, werden Probleme durch Abrasion vermieden.

Wird das Röntgenkontrastmittel als anorganische Nanopartikel in das (Co)polymer eingebracht, so hat es sich als zweckmäßig erwiesen, dass die anorganischen Nanopartikel oberflächenmodifiziert sind, vorzugsweise mit geeigneten, dem Fachmann bekannten Silanen. Die Nanopartikel können sphärisch, ellipsoid, plättchenförmig oder unregelmäßig geformt sein. Die anorganischen Nanopartikel bestehen im Wesentlichen aus ZrO₂.

Nachfolgend werden Ausführungsbeispiele der Erfindung beschrieben.

Eine erfindungsgemäße Knochenzementmischung mit anorganischen Nanopartikeln der Teilchengröße 3-15 nm erhält man beispielsweise, indem man als Monomerkomponente eine für Knochenzemente übliche Monomerkomponente verwendet, und eine Polymerkomponente mit (Co)polymerteilchen mit darin dispergierten röntgenopaken anorganischen Nanopartikeln einsetzt.

Die (Co)polymerteilchen mit darin dispergierten röntgenopaken anorganischen Nanopartikeln werden zuvor hergestellt, indem man Monomere in Gegenwart von beschichteten oder unbeschichteten ZrO₂-Partikeln der Teilchengröße 7 nm suspensionspolymerisiert, Man erhält kugelförmige Polymer- oder Copolymerteilchen mit darin dispergierten röntgenopaken anorganischen Nanopartikeln, Der Anteil an ZrO₂-Partikeln, die in dem erhaltenen (Co)polymer dispergiert vorliegen, beträgt z.B. 15 %. Die Polymerkomponente für den Knochenzement wird nun hergestellt aus den zuvor beschriebenen kugelförmigen Polymer- oder Copolymerteilchen mit darin dispergierten röntgenopaken anorganischen Nanopartikeln und einem Initiator, der im Anteil von etwa 1 % an der Polymerkomponente vorliegt.

In einem weiteren Beispiel für eine Knochenzementmischung mit anorganischen Nanopartikeln der Teilchengröße 3-15 nm wird eine für einen Knochenzement übliche Monomerkomponente verwendet. Die Polymerkomponente besteht aus (Co)polymerteilchen mit darin dispergierten röntgenopaken anorganischen Nanopartikeln und aus kugelförmigen Polymer- oder Copolymerteilchen ohne Röntgenkontrastmittel und aus einem Initiator.

Die (Co)polymerteilchen mit darin dispergierten röntgenopaken anorganischen Nanopartikeln werden zuvor hergestellt indem man übliche Monomere in Gegenwart von beschichteten oder unbeschichteten ZrO₂-Partikeln der Teilchengröße 7 nm suspensionspolymerisiert. Man erhält kugelförmige Polymer- oder Copolymerteilchen mit darin dispergierten röntgenopaken anorganischen Nanopartikeln. Der Anteil an ZrO₂-Partikeln, die in dem erhaltenen (Co)polymer dispergiert vorliegen, beträgt z.B. 30 %. Die Polymerkomponente für den Knochenzement wird nun hergestellt aus den zuvor beschriebenen kugelförmigen Polymer- oder Copolymerteilchen mit darin dispergierten röntgenopaken anorganischen Nanopartikeln mit einem Anteil von 50 % und kugelförmigen Polymer- oder Copolymerteilchen ohne Röntgenkontrastmittel mit einem Anteil von 49 % und einem Initiator, der im Anteil von etwa 1% an der Polymerkomponente vorliegt. Durch das Hinzufügen von nicht röntgenopakem (Co)polymer lässt sich die Röntgenopazität der Knochenzementmischung so einstellen, dass die Röntgenopazität einer Menge von 5-45 %, vorzugsweise 8-16 %, Röntgenkontrastmittel in der Polymerkomponente des Knochenzements entspricht.

## Patentansprüche

1. Knochenzementmischung, gebildet aus einer ein Röntgenkontrastmittel enthaltenden Polymerkomponente und einer Monomerkomponente, **dadurch gekennzeichnet, dass** das Röntgenkontrastmittel vorliegt
als im Wesentlichen kugelförmige Polymer- oder Copolymerteilchen mit darin dispergierten röntgenopaken anorganischen Nanopartikeln der Teilchengröße 3-15 nm, hergestellt durch Suspensionspolymerisation in Gegenwart der Nanopartikel, wobei die Nanopartikel vollständig von dem (Co)polymermaterial bedeckt sind, die anorganischen Nanopartikel im Wesentlichen aus ZrO₂ bestehen und mit Silanen oberflächenmodifiziert sind.

2. Knochenzementmischung nach Anspruch 1, **dadurch gekennzeichnet, dass** die Nanopartikel sphärisch, ellipsoid, plättchenförmig oder unregelmäßig geformt sind.

3. Röntgenkontrastmittel, **dadurch gekennzeichnet; dass** es vorliegt
als im Wesentlichen kugelförmige Polymer oder Copolymerteilchen mit darin dispergierten röntgenopaken anorganischen Nanopartikeln der Teilchengröße 3-15 nm, hergestellt durch Suspensionspolymerisation in Gegenwart der Nanopartikel, wobei die Nanopartikel vollständig von dem (Co)polymermaterial bedeckt sind, und die anorganischen Nanopartikel im Wesentlichen aus ZrO₂ bestehen und mit Silanen oberflächenmodifiziert sind.

## Claims

1. Bone cement mixture, formed from a polymer component containing an X-ray contrast medium, and a monomer component, **characterised in that** the X-ray contrast medium is provided
as essentially sphere-shaped polymer or copolymer particles with radio-opaque inorganic nano-particles of a particle size of 3-15 nm dispersed therein, produced by suspension polymerisation in the presence of nano-particles, whereby the nano-particles are covered completely by the (co)polymer material, and the inorganic nano-particles essentially consist of ZrO₂ and are surface-modified by silanes.

2. Bone cement mixture according to claim 1, **characterised in that** the nano-particles are spherical, ellipsoidal, plate-shaped or irregularly-shaped.

3. X-ray contrast medium, **characterised in that** it is provided as essentially sphere-shaped polymer or copolymer particles with radio-opaque inorganic nano-particles of a particle size of 3-15 nm dispersed therein, produced by suspension polymerisation in the presence of nano-particles, whereby the nano-particles are covered completely by the (co)polymer material, and the inorganic nano-particles essentially consist of ZrO₂ and are surface-modified by silanes.

## Revendications

1. Mélange de ciment osseux formé à partir d'un constituant polymère contenant un agent de contraste radiographique et d'un constituant monomère, **caractérisé en ce que** l'agent de contraste radiographique est présent essentiellement dans la forme de particules polymères ou copolymères de forme sphérique avec des nanoparticules inorganiques opaques à la radiographie, dispersées dans celles-ci, de la taille de particules 3-15 nanomètres, préparé par polymérisation en suspension en présence des nanoparticules, les nanoparticules étant complètement recouvertes du matériau co-polymère, les nanoparticules inorganiques étant essentiellement constituées de ZrO₂ et étant modifiées en surface avec des silanes.

2. Mélange de ciment osseux selon la revendication 1, **caractérisé en ce que** les nanoparticules sont de forme sphérique, de forme ellipsoïdale, de forme de petites plaques ou de forme irrégulière.

3. Agent de contraste radiographique **caractérisé en ce qu'**il est présent essentiellement dans la forme de particules polymères ou copolymères de forme sphérique avec des nanoparticules inorganiques opaques à la radiographie, dispersées dans celles-ci, de la taille de particules 3-15 nanomètres, préparé par polymérisation en suspension en présence des nanoparticules, les nanoparticules étant complètement recouvertes du matériau co-polymère et les nanoparticules inorganiques étant essentiellement constituées de ZrO₂ et étant modifiées en surface avec des silanes.
